Europäisches Patentamt

European Patent Office

Office européen des brevets

(19)

(11) Numéro de publication: **0 148 070**
**B1**

(12) **FASCICULE DE BREVET EUROPEEN**

(45) Date de publication du fascicule du brevet: **13.07.88**

(51) Int. Cl.⁴: **C 07 C 7/04, C 10 G 5/06**

(21) Numéro de dépôt: **84402621.1**

(22) Date de dépôt: **18.12.84**

(54) Procédé et installation de recupération des hydrocarbures les plus lourds d'un mélange gazeux.

(30) Priorité: **30.12.83 FR 8321062**

(43) Date de publication de la demande:
**10.07.85 Bulletin 85/28**

(45) Mention de la délivrance du brevet:
**13.07.88 Bulletin 88/28**

(84) Etats contractants désignés:
**AT BE CH DE FR GB IT LI LU NL SE**

(56) Documents cités:
**DE-A- 293 561**
**US-A-3 397 138**

(73) Titulaire: **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cédex 07 (FR)**

(72) Inventeur: **Gauthier, Pierre**
**95, bd Jean Jaurès**
**F-94260 Fresnes (FR)**

(74) Mandataire: **Jacobson, Claude et al**
**L'AIR LIQUIDE, SOCIETE ANONYME POUR L'ETUDE ET L'EXPLOITATION DES PROCEDES GEORGES CLAUDE 75, quai d'Orsay**
**F-75321 Paris Cédex 07 (FR)**

Courier Press, Leamington Spa, England.

## Description

La présente invention est relative à un procédé de récupération des hydrocarbures les plus lourds d'un mélange gazeux, notamment de récupération d'un mélange de propane, de butane et de pentane à partir d'un gaz résiduaire de raffinerie de pétrole, du type dans lequel on refroidit le gaz à traiter, on le condense partiellement et on distille le condensat.

Les mélanges de propane, butane et pentane, généralement appelés gaz de pétrole liquéfiés ou GPL, se condensent à température ambiante sous une pression de l'ordre de 10 à 20 bars. Leur récupération dans les gaz résiduaires des raffineries de pétrole est un fait récent lié à l'évolution des facteurs économiques.

Dans les gaz résiduaires des raffineries, les GPL sont dilués avec des constituants plus légers, tels que l'éthane, le méthane et l'hydrogène. Il est en général nécessaire de débarrasser ces gaz, dans une mesure prédéterminée, de ces constituants légers avant de les envoyer à une unité de traitement de GPL qui en sépare les constituants par distillation. C'est pourquoi le procédé de récupération comprend lui-même une opération de distillation.

Un tel procédé de récupération nécessite, pour atteindre un bon rendement d'extraction en propane, butane et pentane, un refroidissement du gaz résiduaire jusqu'à une température de l'ordre de −80°C. Or, l'obtention de ce niveau de température par utilisation d'un groupe frigorifique conduit à faire intervenir des groupes frigorifiques complexes à deux étages, à vaporisation sous vide, ce qui pose des problèmes d'investissement et d'exploitation.

Le DE—A—29 32 561 décrit un procédé de récupération des hydrocarbures les plus lourds d'un mélange gazeux, dans lequel la température basse est obtenue grâce à un condenseur fonctionnant à cette température.

La présente invention a pour but de fournir un procédé permettant d'obtenir un rendement d'extraction du même ordre d'une façon particulièrement simple et économique.

A cet effet, l'invention a pour objet un procédé du type précité, caractérisé en ce qu'on vaporise à contre-courant du gaz à traiter une partie du liquide condensé en tête de la colonne de distillation.

Dans des modes de mise en oeuvre avantageux de l'invention:

— on sous-refroidit le liquide condensé en tête de colonne jusqu'à la température finale de refroidissement du gaz à traiter et on la détend avant de le vaporiser;

— on réunit audit liquide détendu la vapeur de tête de la colonne détendue à la même pression et/ou la vapeur issue du gaz à traiter refroidi, après avoir fait circuler celle-ci à contre-courant de ce gaz et l'avoir détendue dans une turbine.

L'invention a également pour objet une installation destinée à la mise en oeuvre d'un tel procédé. Cette installation, du type comprenant une ligne d'échange thermique pourvue de moyens de séparation du condensat du gaz à traiter refroidi, et une colonne de distillation dans laquelle est introduit ce condensat, est caractérisée en ce que la colonne de distillation comprend des moyens de récupération d'une partie du liquide condensé en tête de colonne et des moyens pour faire circuler le liquide récupéré à contre-courant du gaz à traiter dans la ligne d'échange thermique.

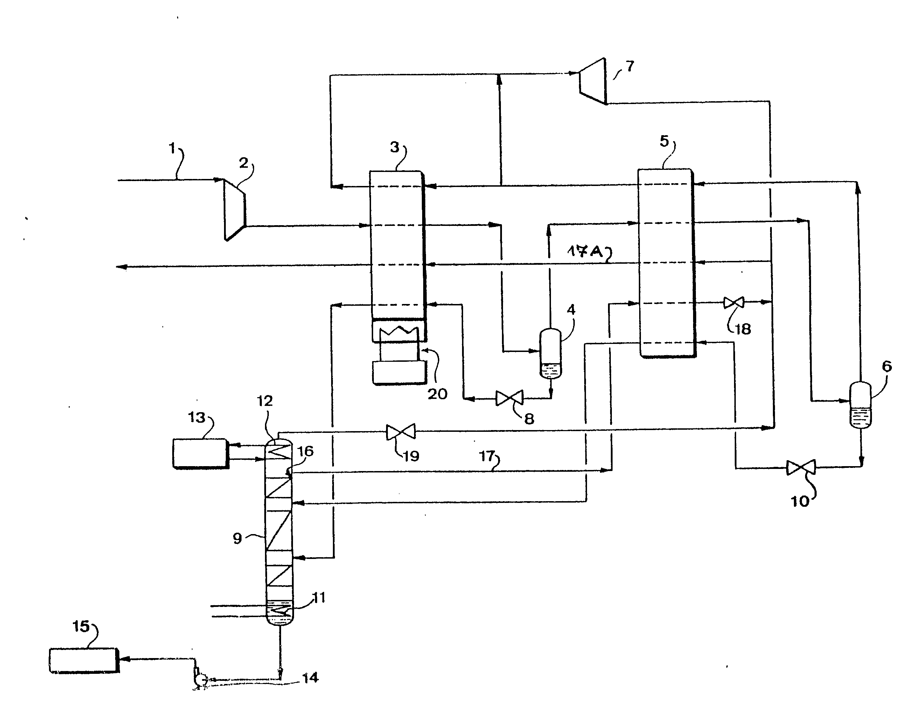

Un exemple de réalisation de l'invention va maintenant être décrit en regard du dessin annexé, dont la figure unique représente schématiquement une installation de récupération de GPL conforme à l'invention.

Le gaz à traiter, acheminé par une conduite 1, est un gaz résiduaire de raffinerie de pétrole constitué d'un mélange d'hydrocarbures de C1 à C5 et d'hydrogène. Le traitement a pour but d'éliminer presque totalement les constituants légers (hydrocarbures en C1 et C2 et hydrogène) de ce mélange. Toutes les pressions indiquées ci-dessous sont des pressions absolues.

Le gaz, comprimé à 23 bars, 30°C dans un compresseur 2, est refroidi jusqu'à une température intermédiaire de l'ordre de −30°C et partiellement condensé dans un échangeur de chaleur 3, puis séparé en une phase liquide et une phase vapeur dans un séparateur de phases 4. La phase vapeur est refroidie jusqu'à −82°C environ dans un deuxième échangeur de chaleur 5, puis séparée en une phase liquide et une phase vapeur dans un second séparateur de phases 6.

La vapeur issue du séparateur 6, presque uniquement constituée d'éthane, de méthane et d'hydrogène, est envoyée à contre-courant dans l'échangeur 5; une partie de ce courant est dérivé vers une turbine 7, tandis que le reste traverse à contre-courant l'échangeur 3 puis est également envoyé à cette turbine. Le gaz détendu à 6 bars dans la turbine 7 traverse les échangeurs 5 et 3 à contre-courant et constitue une partie du gaz résiduaire de l'installation de récupération.

La fraction liquide issue du séparateur 4 est détendu dans une vanne de détente 8, traverse à contre-courant l'échangeur 3 puis est introduite dans une colonne de distillation 9 maintenue à une pression de 17 bars. De même, la fraction liquide issue du séparateur 6 est détendue dans une vanne de détente 10, traverse à contre-courant l'échangeur 5 puis est introduite dans la colonne 9, à un niveau supérieur à celui correspondant à l'autre fraction liquide.

La colonne 9 est équipée en cuve d'un échangeur de chaleur 11 de chauffage dans lequel circule un fluide calorigène qui peut être de la vapeur d'eau à une pression voisine de la pression atmosphérique, et en tête d'un échangeur de chaleur 12 condenseur relié à un groupe frigorifique 13, par exemple à ammoniac ou à "Fréon", fournissant des frigories à −33°C. Le liquide de cuve de la colonne 9, comprimé à 25 bars par une pompe 14, est envoyé dans un réservoir de stockage 15 en vue d'un traitement ultérieur; il est constitué d'un mélange de propane, de butane et de pentane débarrassé dans une mesure prédéterminée des constituants plus légers et contient par exemple de l'éthane dans une proportion inférieure à 2%.

Une partie du liquide condensé par le condenseur 12, constitué essentiellement d'éthane et de méthane, est récupérée par une auge 16 et est envoyée par une conduite 17 à l'échangeur 5, dans lequel il est sous-refroidi. A l'extrémité froide de cet échangeur, ce liquide est détendu à 6 bars dans une vanne de détente 18 et réuni au courant gazeux sortant de la turbine 7. De même, la vapeur de tête de la colonne 9, constituée essentiellement, comme le liquide condensé par le condenseur 12, d'éthane et de méthane, est détendue à 6 bars dans une vanne de détente 19 puis réunie au bout froid de l'échangeur 5 au courant gazeux sortant de la turbine 7. Ainsi, c'est l'ensemble du courant gazeux turbiné et des courants détendus dans les vannes 18 et 19 qui traverse à contre-courant les échangeurs 5 puis 3 via une conduite 17A pour produire du froid puis constituer le gaz résiduaire de l'installation de récupération.

On comprend donc que l'installation décrite ci-dessus permet d'obtenir le niveau de température de −80°C nécessaire à un bon rendement d'extraction en GPL en utilisant un simple groupe frigorifique à un étage fonctionnant vers −30 à −40°C.

De préférence, la puissance frigorifique à −33°C nécessaire pour le fonctionnement de l'installation est répartie entre la tête de la colonne 9 et l'échangeur 3. C'est ce qu'on a illustré sur le dessin en combinant à cet échangeur un second groupe frigorifique 20. En fait, en pratique, l'installation comporte un groupe frigorifique unique dont une partie du fluide réfrigérant parcourt l'échangeur 12 de tête de colonne et une autre partie est envoyée dans des passages à contre-courant de l'échangeur 3.

On a représenté sur le dessin deux échangeurs de chaleur distincts 3 et 5. En pratique, on peut avantageusement utiliser un échangeur de chaleur unique du type à plaques brasées, pourvu des entrées et sorties de fluides nécessaires.

On comprend que le même principe de transfert de frigories à un niveau de température inférieur par le liquide de tête de la colonne de distillation peut être appliqué à d'autres types de récupérations de mélanges d'hydrocarbures ayant un nombre d'atomes de carbone au moins égal à un nombre donné, à partir d'un mélange gazeux contenant, outre ces hydrocarbures, des constituants plus volatils.

## Revendications

1. Procédé de récupération des hydrocarbures les plus lourds d'un mélange gazeux, notamment de récupération d'un mélange de propane, de butane et de pentane à partir d'un gaz résiduaire de raffinerie de pétrole, du type dans lequel on refroidit le gaz à traiter, on le condense partiellement et on distille le condensat, caractérisé en ce qu'on vaporise à contre-courant du gaz à traiter une partie du liquide condensé en tête de la colonne de distillation (9).

2. Procédé suivant la revendication 1, caractérisé en ce qu'on sous-refroidit le liquide condensé en tête de colonne jusqu'à la température finale de refroidissement du gaz à traiter et on le détend avant de la vaporiser.

3. Procédé suivant la revendication 2, caractérisé en ce qu'on réunit le liquide détendu à la vapeur de tête de la colonne (9) détendue à la même pression.

4. Procédé suivant l'une des revendications 2 et 3, caractérisé en ce qu'on fait circuler à contre-courant du gaz à traiter la vapeur issue de ce gaz refroidi, on détend cette vapeur dans une turbine (7) et on la réunit audit liquide détendu.

5. Installation de récupération des hydrocarbures les plus lourds d'un mélange gazeux, notamment de récupération d'un mélange de propane, de butane et de pentane à partir d'un gaz résiduaire de raffinerie de pétrole, du type comprenant une ligne d'échange thermique (3 à 6) pourvue de moyens (4, 6) de séparation du condensat du gaz à traiter refroidi, et une colonne de distillation (9) dans laquelle est introduit ce condensat, caractérisée en ce que la colonne de distillation (9) comprend des moyens (16) de récupération d'une partie du liquide condensé en tête de colonne et des moyens (17A) pour faire circuler le liquide récupéré à contre-courant du gaz à traiter dans la ligne d'échange thermique.

6. Installation suivant la revendication 5, caractérisée en ce que lesdits moyens comprennent des passages de sous-refroidissement du liquide condensé en tête de colonne, prévus dans la ligne d'échange thermique (3 à 6), une vanne de détente (18) et des passages pour le fluide ainsi détendu à contre-courant du gaz à traiter, également prévus dans la ligne d'échange thermique.

7. Installation suivant la revendication 6, caractérisée en ce qu'elle comprend des moyens (19) pour détendre la vapeur de tête de la colonne de distillation et des moyens pour réunir cette vapeur détendue au fluide sortant de ladite vanne de détente (18).

8. Installation suivant l'une des revendications 6 et 7, caractérisée en ce que la ligne d'échange thermique comprend un séparateur de phases final (6) pour le gaz à traiter refroidi, des passages à contre-courant du gaz à traiter pour la vapeur issue de ce séparateur, une turbine (7) de détente de cette vapeur, et des moyens pour réunir cette vapeur détendue au fluide sortant de ladite vanne de détente (18).

9. Installation suivant l'une quelconque des revendications 5 à 8, caractérisée en ce que la ligne d'échange thermique comprend deux tronçons d'échange de chaleur (3, 5) en série et un séparateur de phases 4, 6) après chacun de ces tronçons.

## Patentansprüche

1. Verfahren zur Rückgewinnung der schwersten Kohlenwasserstoffe eines gasförmigen Gemisches, insbesondere zur Rückgewinnung eines Gemisches von Propan, Butan und Pentan aus einem Restgas einer Erdölraffinerie, bei welchem man das zu behandelnde Gas kühlt, es teilweise kondensiert und das Kondensat destilliert, dadurch gekennzeichnet, daß man im Gegenstrom zu zu behandelndem Gas einen Teil der am Kopf der Destillationskolonne (9) kondensierten Flüssigkeit verdampft.

2. Verfahren nach Anspruch 1, dadurch gekennzeichnet, daß man die am Kopf der Kolonne kondensierte Flüssigkeit bis zu der Endtemperatur der Kühlung des zu behandelnden Gases unterkühlt und vor seiner Verdampfung entspannt.

3. Verfahren nach Anspruch 2, dadurch gekennzeichnet, daß man die entspannte Flüssigkeit mit dem Kopfdampf der Kolonne (9), der auf den gleichen Druck entspannt wurde, vereinigt.

4. Verfahren nach einem der Ansprüche 2 und 3, dadurch gekennzeichnet, daß man im Gegenstrom zu zu behandelndem Gas den aus diesem gekühlten Gas stammenden Dampf zirkulieren läßt, diesen Dampf in einer Turbine (7) entspannt und mit der besagten entspannten Flüssigkeit vereinigt.

5. Vorrichtung zur Rückgewinnung der schwersten Kohlenwasserstoffe eines gasförmigen Gemisches, insbesondere zur Rückgewinnung eines Gemisches von Propan, Butan und Pentan aus einem Restgas einer Erdölraffinerie, mit einer Wärmeaustauschlinie (3 bis 6), die mit Einrichtungen (4, 6) zur Abtrennung von Kondensat von dem gekühlten zu behandelnden Gas versehen ist, und einer Destillationskolonne (9), in welche dieses Kondensat eingeführt wird, dadurch gekennzeichnet, daß die Destillationskolonne (9) Einrichtungen (16) zur Rückgewinnung eines Teils der am Kopf der Kolonne kondensierten Flüssigkeit und Einrichtungen (17A) zum Zirkulierenlassen der rückgewonnenen Flüssigkeit im Gegenstrom zu zu behandelndem Gas in der Wärmeaustauschlinie umfaßt.

6. Vorrichtung nach Anspruch 5, dadurch gekennzeichnet, daß die Einrichtungen Unterkühlungsdurchgänge für die am Kopf der Kolonne kondensierte Flüssigkeit, die in der Wärmeaustauschlinie (3 bis 6) vorgesehen sind, ein Druckminderventil (18) und Durchgänge für das somit entspannte Fließmittel im Gegenstrom zu zu behandelndem Gas, die ebenfalls in der Wärmeaustauschleitung vorgesehen sind, aufweisen.

7. Vorrichtung nach Anspruch 6, dadurch gekennzeichnet, daß sie Einrichtungen (19) zum Entspannen des Kopfdampfes der Destillationskolonne und Einrichtungen zur Vereinigung dieses entspannten Dampfes mit Fließmittel, das das Druckminderventil (18) verläßt, aufweist.

8. Vorrichtung nach einem der Ansprüche 6 und 7, dadurch gekennzeichnet, daß die Wärmeaustauschlinie eine Endphasentrenneinrichtung (6) für das gekühlte zu behandelnde Gas, Durchgänge für den aus dieser Trenneinrichtung stammenden Dampf im Gegenstrom zu zu behandelndem Gas, eine Turbine (7) zur Entspannung dieses Dampfes und Einrichtungen zur Vereinigung dieses entspannten Dampfes mit das Druckminderventil (18) verlassendem Fließmittel aufweist.

9. Vorrichtung nach einem der Ansprüche 5 bis 8, dadurch gekennzeichnet, daß die Wärmeaustauschlinie zwei Wärmeaustauschabschnitte (3, 5) in Reihe sowie eine Phasentrenneinrichtung (4, 6) nach jedem dieser Abschnitte aufweist.

**Claims**

1. Method of recovering the heaviest hydrocarbons from a gaseous mixture, particularly for recovery of a mixture of propane, butane and pentane from a residual petroleum refinery gas, of the type in which the gas to be treated is cooled and partly condensed and the condensate is distilled, characterised in that a part of the liquid condensed at the top of the distillation column (9) is vapourised in counter-current with the gas to be treated.

2. Method according to claim 1, characterised in that the liquid condensed at the top of the column is subcooled to the final cooling temperature of the gas to be treated and is expanded before being vapourised.

3. Method according to claim 2, characterised in that the expanded liquid is combined with the vapour from the top of the column (9) expanded to the same pressure.

4. Method according to one of the claims 2 and 3, characterised in that the vapour coming from this cooled gas is caused to flow in counter-current with the gas to be processed, and this vapour is expanded in a turbine (7) and combined with the said expanded liquid.

5. Installation for recovery of the heaviest hydrocarbons from a gaseous mixture, particularly for recovery of a mixture of propane, butane and pentane from a residual petroleum refinery gas, of the kind comprising a heat exchange line (3 to 6) provided with means (4, 6) for separating the condensate of the cooled gas to be treated and a distillation column (9) into which this condensate is introduced, characterised in that the distillation column (9) comprises means (16) for recovering a part of the condensed liquid at the top of the column and means (17A) for causing the recovered liquid to circulate in counter-current with the gas to be treated in the heat exchange line.

6. Installation according to claim 5, characterised in that the said means comprise subcooling passages for the liquid condensed at the top of the column, provided in the heat exchange line (3 to 6), an expansion valve (18) and passages for the fluid thus expanded in counter-current with the gas to be treated, also provided in the heat exchange line.

7. Installation according to claim 6, characterised in that it comprises means (19) for expanding the vapour from the top of the distillation column and means for combining this expanded vapour with the fluid issuing from the said expansion valve (18).

8. Installation according to one of the claims 6 and 7, characterised in that the heat exchange line comprises a final phase separator (6) for the cooled gas to be treated, counter-current passages of the gas to be treated for the vapour emerging from this separator, a turbine (7) for expansion of this vapour, and means for combining this expanded vapour with the fluid emerging from the said expansion valve (18).

9. Installation according to any one of the claims 5 to 8, characterised in that the heat exchange line comprises two heat exchange sections (3 to 6) in series, and a phase separator (4, 6) after each of these sections.